# EUROPEAN PATENT SPECIFICATION

(11) **EP 1 622 646 B1**
(45) Date of publication and mention of the grant of the patent: **08.08.2012**
(21) Application number: 04751467.4
(22) Date of filing: 06.05.2004
(51) Int. Cl.: A61K 47/48, A61P 37/00

(54) **TREATMENT OF LUPUS TARGETING THE MACROPHAGES OR THE FOLATE RECEPTOR**
Behandlung von Lupus mit den Makrophagen oder dem Folat-Rezeptor als Target
Traitement du lupus par ciblage des macrophages ou du recepteur de folate

(30) Priority: 06.05.2003 US 468330 P
(43) Date of publication of application: 08.02.2006
(73) Proprietor: Purdue Research Foundation, West Lafayette, IN 47906 (US)
(72) Inventor: LOW, Philip, Stewart, West Lafayette, IN 47906 (US); VARGHESE, Bindu, West Lafayette, IN 47906 (US)
(74) Representative: Elsy, David
(86) International application number: PCT/US2004/014097
(87) International publication number: WO 2004/100983

(56) References cited:
- WO-A-01/74382
- WO-A-96/36367
- WO-A-98/58678
- WO-A-99/41285
- WO-A-02/087424
- WO-A-03/092742
- REDDY J A ET AL: "FOLATE-MEDIATED TARGETING OF THERAPEUTIC AND IMAGING AGENTS TO CANCERS" CRITICAL REVIEWS IN THERAPEUTIC DRUG CARRIER SYSTEMS, vol. 15, no. 6, 1998, pages 587-627, XP000901554 ISSN: 0743-4863
- NAKASHIMA-MATSUSHITA N ET AL: "Selective expression of folate receptor beta and its possible role in methotrexate transport in synovial macrophages from patients with rheumatoid arthritis" ARTHRITIS AND RHEUMATISM, LIPPINCOTT, PHILADELPHIA, US, vol. 42, no. 8, August 1999 (1999-08), pages 1609-1616, XP002284073 ISSN: 0004-3591
- TURK M J ET AL: "Folate-targeted imaging of activated macrophages in rats with adjuvant-induced arthritis" ARTHRITIS AND RHEUMATISM, LIPPINCOTT, PHILADELPHIA, US, vol. 46, no. 7, July 2002 (2002-07), pages 1947-1955, XP004536129 ISSN: 0004-3591

## Description

### FIELD OF THE INVENTION

This invention relates to a conjugate for treating lupus erythematosus. More particularly, ligands that hind to activated macrophages or other stimulated immune cells are conjugated with an immunogen, a cyotoxin, or another agent for altering macrophage function, or the function of other stimulated immune cells, for administration to a patient for treatment of lupus erythematosus.

### BACKGROUND AND SUMMARY OF THE INVENTION

The mammalian immune system provides a means for the recognition and elimination of foreign pathogens. While the immune system normally provides a line of defense against foreign pathogens, there are many instances where the immune response itself is involved in the progression of disease. Exemplary of diseases caused or worsened by the host's own immune response are autoimmune diseases such as multiple sclerosis, lupus erythematosus, psoriasis, pulmonary fibrosis, and rheumatoid arthritis and diseases in which the immune response contributes to pathogenesis such as atherosclerosis, inflammatory diseases, osteomyelitis, ulcerative colitis, Crohn's disease, and graft versus host disease often resulting in organ transplant rejection.

Macrophages are generally the first cells to encounter foreign pathogens, and accordingly, they play an important role in the immune response. Activated macrophages nonspecifically engulf and kill foreign pathogens within the macrophage by hydrolytic and oxidative attack resulting in degradation of the pathogen. Peptides from degraded proteins are displayed on the macrophage cell surface where they can be recognized by T cells, and they can directly interact with antibodies on the B cell surface, resulting in T and B cell activation and further stimulation of the immune response.

Lupus erythematosus, including both systemic and cutaneous forms of the disease, is an autoimmune disease of unknown etiology in which tissues are damaged by autoantibodies and immune complexes. There is evidence that there is a genetic predisposition to lupus erythematosus, and the prevalence of this disease is about eight times higher in women of child-bearing age than in men. There are also environmental factors that may trigger episodes of lupus erythematosus including ingestion of some foods and exposure to certain chemicals and ultraviolet light. The prevalence of systemic lupus erythematosus in urban areas is about 15-50 for a population of 100,000.

Abnormal immune responses that are known to be involved in systemic lupus erythematosus include aberrant regulation of self-reactive T and B lymphocytes resulting in increased activity of these cells. This T and B cell hyperactivity permits sustained production of autoantibodies and immune complexes. Some of the autoantibodies produced induce the symptoms of lupus erythematosus by binding directly to self-antigens and others attach to cell membranes via charge interactions or via cross-reactivity with cell or tissue components. Accordingly, not only is the etiology of lupus erythematosus unknown, but, except for the involvement of T and B cells in lupus erythematosus, the involvement of other immune cells in lupus erythematosus is not well-understood.

There are multiple clinical manifestations of lupus erythematosus including musculoskeletal manifestations, such as myalgias and intermittent arthritis, cutaneous manifestations, including erythematosus rashes, renal manifestations such as deposition of immunoglobulins in the glomeruli and clinical nephritis, nervous system manifestations such as mild cognitive dysfunction, headaches, and seizures, vascular and hematologic symptoms including thrombosis, anemia, hemolysis, leukopenia, and thrombocytopenia, cardiopulmonary manifestations such as pericarditis, arrhythmias, pleurisy, and pleural effusions, gastrointestinal manifestations such as nausea and diarrhea, and ocular manifestations such as retinal vasculitis. Treatments for lupus erythematosus include the use of glucocorticoids and non-steroidal anti-inflammatory drugs (NSAIDs) and daily treatment with antimalarials, such as hydroxychloroquine, is used to improve skin lesions. However, glucocorticoids and NSAIDs, in particular, have undesirable side effects and attempts are made to limit their use to minimize undesirable side effects. Therefore, the available therapies for the treatment of the multiple clinical manifestations of lupus erythematosus have undesirable side effects highlighting the need for new therapies to treat this disease.

W002087424 discloses a method of treating or monitoring/diagnosing a disease state mediated by activated macrophages. The method comprises the step of administering to a patient suffering from a macrophage mediated disease state an effective amount of a composition comprising a conjugate or complex of the general formula: Ab-X, where the group Ab comprises a ligand capable of binding to activated macrophages, and when the conjugate is being used for treatment of the disease state, the group X comprises an immunogen, a cytotoxin, or a compound capable of altering macrophage function, and when the conjugate is being used for monitoring/diagnosing the disease state, X comprises an imaging agent. The method is said to be useful for treating a patient suffering from a disease selected from the group consisting of rheumatoid arthritis, ulcerative colitis, Crohn's disease, inflammation, infections, osteomyelitis, atherosclerosis, organ transplant rejection, pulmonary fibrosis, sarcoidosis, and systemic sclerosis.

WO9941285 discloses methods and compositions for selectively targeting macrophages in a localized area. The compositions of the invention include an Fc receptor binding agent, and a toxic or a detectable agent. Methods for depleting or inhibiting the activity of macrophages using the compositions of the invention are disclosed. The compositions of the invention can be used therapeutically and diagnostically.

WO9636367 discloses a method for enhancing transmembrane transport of a diagnostic agent across a membrane of a living cell. The method comprises contacting a membrane of a living cell with a complex formed between said diagnostic agent and ligands selected from biotin or biotin receptor-binding analogs of biotin, folate or folate receptor-binding analogs of folate, riboflavin or riboflavin receptor-binding analogs of riboflavin to initiate receptor mediated transmembrane transport of the ligand complex. The method may be used for imaging tissues in vivo.

WO0174382 discloses a method and pharmaceutical composition is provided for enhancing the endogenous immune response-mediated elimination of a population of pathogenic cells in a host animal wherein the pathogenic cells preferentially express,uniquely express, or over express a binding site for a particular ligand. The invention comprises administering the ligand conjugated to an immunogen to a host animal harboring the population of pathogenic cells. Antibodies, preexisting or administered to the host animal to establish a passive immunity, directed against the immunogen bind to the ligand-immunogen conjugate resulting in elimination of the pathogenic cell by the host's immune response. At least one additional therapeutic factor is administered selected from the group consisting of a cell killing agent, a tumor penetration enhancer, a chemotherapeutic agent, antimicrobial agent, a cytotoxic immune cells, and a compound capable of stimulating an endogenous immune response wherein the compound does not bind to the ligand-immunogen conjugate.

Reddy et al ((1998) Critical Reviews in Therapeutic Drug Carrier Systems, 15(6): 587-627) discloses the use of folate-mediated tumor targeting for delivery of the following molecules and molecular complexes to tumor tissue: (i) protein toxins, (ii) low-molecular-weight chemotherapeutic agents, (iii) radioimaging agents, (iv) MRI contrast agents, (v) radiotherapeutic agents, (vi) liposomes with entrapped drugs, (vii) genes, (viii) antisense oliogonucleotides, (ix) ribozymes, and (x) immunotherapuetic agents. Reddy et al indicate that in virtually all cases, *in vitro* studies demonstrate a significant improvement in potency and/or cancer-cell specificity over the nontargeted form of the same pharmaceutical agent.

Nakashima-Matsushita et al ((1999) Arthritis & Rheumatism, 42(8): 1609-1616) discloses that folate receptor FR-β expression is selectively elevated in rheumatoid arthritis (RA) synovial macrophages and suggest that methotrexate is transported through FR-β in RA synovial macrophages. Nakashima-Matsushita therefore suggests that folate antagonists with higher affinity for FR-β would be useful in the treatment of RA.

Turk et al ((2002) Arthritis & Rheumatism, 46(7): 1947-1955) discloses that folic acid conjgated to a ^{99m}Tc chelator may be useful for assaying the participation of activated macrophages in inflammatory processes such as rheumatoid arthritis.

The present invention is directed to the use of a conjugate of the general formula L-X where the group L comprises a ligand capable of binding to activated macrophages and the group X comprises an immunogen, a cytotoxin, or another compound capable of altering macrophage function, in the manufacture of a medicament for treating lupus erythematosus. In another embodiment, L can comprise a vitamin, or a vitamin-receptor binding analog or derivative thereof.

The conjugate may be used in a method for treating lupus erythematosus by killing or inhibiting the function of activated macrophages or other stimulated immune cells. The host immune response may be redirected to activated macrophages by "labeling" activated macrophages with an immunogen. To promote killing or to inhibit the function of activated macrophages, ligands that bind preferentially to activated macrophages compared to resting macrophages may be conjugated to a cytotoxin for direct killing or inhibition of the function of activated macrophages. Ligands that bind preferentially to activated macrophages may be conjugated to another compound capable of altering the function of activated macrophages to inhibit activity of the activated macrophage population. Ligands that can be used in the conjugates of the present invention include those that bind to receptors preferentially expressed or presented on activated macrophages compared to resting marcophages, such as the folate receptor, or ligands such as monoclonal antibodies directed to cell surface markers preferentially expressed on activated macrophages.

Treating lupus erythematosus may comprise the step of administering to a patient suffering from lupus erythematosus an effective amount of a composition comprising a conjugate of the general formula L-X where the group L comprises a ligand capable of binding to activated macrophages and the group X comprises an immunogen, a cytotoxin, or another compound capable to altering macrophage function.

Treating lupus erythematosus may comprise the step of administering to a patient suffering from lupus erythematosus an effective amount of a composition comprising a conjugate of the general formula L-X where the group L comprises a vitamin, or a vitamin-receptor binding analog or derivative therof, and the group X comprises an immunogen, a cytotoxin, or another compound capable to altering macrophage function.

### BRIEF DESCRIPTION OF THE DRAWINGS

Fig. 1 shows the percentage uptake of EC2O in the organs of lupus prone MRL/lpr mice (bar on left for each organ) versus control mice (bar on right for each organ).
Fig. 2 shows a photomicrograph of lupus prone MRL/lpr mice that have either been injected with the ligand conjugates of the present invention (mouse on the left) or have been injected with PBS (control; mouse on the right).
Fig. 3 shows a survival curve for lupus prone MRL/lpr mice that have been injected with ligand conjugates used in the method of the present invention, folate-FITC (open diamonds), or have been injected with PBS (control; closed diamonds).

### DETAILED DESCRIPTION OF THE INVENTION

The use in a method is provided for treating lupus erythematosus. Lupus erythematosus can be treated by administering an effective amount of a composition of the formula L-X, wherein L comprises a ligand capable of binding to an activated macrophage and wherein the group X comprises an immunogen, capable of altering macrophage function. Such macrophage targeting conjugates, when administered to a patient suffering from lupus erythematosus, can work to concentrate and associate the conjugated cytotoxin, immunogen, or other compound capable of altering macrophage function with a population of activated macrophages to kill the activated macrophages or alter macrophage function. Elimination or deactivation of the activated macrophage population can work to eliminate or reduce the pathogenesis characteristic of lupus erythematosus. The conjugate is typically administered parenterally as a composition comprising the ligand conjugate and a pharmaceutically acceptable carrier therefor.
Conjugate administration is typically continued until symptoms of the disease state are reduced or eliminated.

The method disclosed herein can be used for both human clinical medicine and veterinary applications. Thus, the patient afflicted with lupus erythematosus, or a similar disease state, can be a human, or in the case of veterinary applications, can be a laboratory, agricultural, domestic or a wild animal. The conjugates are preferably administered parenterally to the patient suffering from lupus erythematosus, for example, intradermally, subcutaneously, intramuscularly, intraperitoneally, or intravenously. Alternatively, the conjugates can be administered to the patient by other medically useful procedures and effective doses can be administered in standard or prolonged release dosage forms, such as a slow pump. The therapeutic method may be used alone, or in combination with other therapeutic methods, including those recognized for the treatment of lupus erythematosus. In general, the ligand conjugates can be formed from a wide variety of ligands, including any ligand capable of binding to a receptor expressed or presented on the surface of activated macrophages that is not expressed/presented or is not present in significant amounts on the surface of resting macrophages. Such ligands include N-formyl peptides (e.g., f-Met-Leu-Phe), high mobility group factor 1 protein (HMGB1), hyaluronan fragments, HSP-70, toll-like receptor ligands, scavenger receptor ligands, co-receptors for antigen presentation, ligands that bind to the CD68, BER-MAC3, RFD7, CD4, CD14, and HLA-D markers on activated macrophages, antibodies, or fragments thereof, that bind preferentially to activated macrophages, and vitamins or receptor-binding vitamin analogs/derivatives. The ligand conjugates are capable of preferentially binding to activated macrophages compared to resting macrophages due to preferential expression of the receptor for the ligand on activated macrophages compared to resting macrophages.

Acceptable vitamin moieties that can be used as ligands in accordance with the invention include folic acid, and their receptor binding analogs and derivatives as defined in the claims. They constitute the targeting entity that can be coupled with an immunogen, capable of altering macrophage function, to form the ligand conjugates for use in accordance with the invention. Vitamin moieties include folic acid, and receptor-binding analogs and derivatives thereof (see U.S. Patent No 5,688,488).

In the ligand conjugates of the general formula L-X in accordance with one embodiment of the present invention, the group L is a ligand capable of binding to activated macrophages as compared to resting macrophages as described above. The activated macrophage binding ligand is folic acid, a folic acid analog or other folate receptor-binding molecules.

Activated macrophages express a 38 kD GPI-anchored folate receptor that binds folate and folate-derivatized compounds with subnanomolar affinity (i.e., < 1 nM). Importantly, covalent conjugation of small molecules, proteins, and even liposomes to folic acid does not alter the vitamin's ability to bind the folate receptor. Because most cells use an unrelated reduced folate carrier to acquire the necessary folic acid, expression of the folate receptor is restricted to a few cell types. With the exception of kidney and placenta, normal tissues express low or nondetectable levels of the folate receptor. However, many malignant tissues, including ovarian, breast, bronchial, and brain cancers express significantly elevated levels of the receptor. In fact, it is estimated that 95% of all ovarian carcinomas overexpress the folate receptor. Also, it has recently been reported that the folate receptor ß, the nonepithetial isoform of the folate receptor, is expressed on activated, but not resting synovial macrophages. Thus, Applicants have found that folate-immunogen conjugates can be used in the method described herein to redirect the host immune response in animals with lupus erythematosus to activated macrophages to deplete macrophages and reduce the symptoms of disease.

Conjugates wherein the group 1. is folic acid, a folic acid analog, or another folic acid receptor-binding ligand are described in detail in U.S. Patent No. 5,688,488. That patent, as well as related U.S. Patents Nos. 5,416,016 and 5,108,921, describe methods and examples for preparing conjugates useful in accordance with the present invention. The present macrophage-targeted ligand conjugates can be prepared and used following general protocols described in those patents.

Folinic acid, pteroic acid, pteropolyglutamic acid, and folate receptor-binding pteridines such as tetrahydropterins, dihydrofolates, tetrahydrofolates, and their deaza and dideaza analogs can also be used in accordance with the invention. The terms "deaza" and "dideaza" analogs refers to the art-recognized folate analogs having a carbon atom substituted for one or two nitrogen atoms in the naturally occurring folic acid structure. For example, the deaza analogs include the 1-deaza, 3-deaza, 5-deaza, 8-deaza, and 10-deaza analogs. The dideaza analogs include, for example, 1,5 dideaza, 5,10-dideaza, 8,10-dideaza, and 5,8-dideaza analogs. The foregoing are folate analogs or derivatives and can bind to folate receptors. Other folate analogs or derivatives useful in accordance with the invention are the folate receptor-binding analogs aminopterin, amethopterin (methotrexate), N¹⁰ - methylfolate, 2-deamino-hydroxyfolate, deaza analogs such as 1-deazamethopterin or 3-deazamethopterin, and 3'5'-dichloro-4-amino-4-deoxy-N¹⁰-methylpteroylglutamic acid (dichloromethotrexate). Another folate analog is folie acid containing a glutamic acid residue in the D configuration (folic acid normally contains one glutamic acid in the L configuration linked to pteroic acid).

The binding site for the ligand can include receptors for any ligand molecule, or a derivative or analog thereof, preferentially expressed/presented on the surface of activated macrophages other stimulated immune cells. A surface-presented protein preferentially expressed by activated macrophages is a receptor that is either not present or is present at insignificant concentrations on resting macrophages providing a means for preferential binding to activated macrophages. Accordingly, any receptor that is upregulated on activated macrophages compared to resting macrophages, or which is not expressed/presented on the surface of resting macrophages, or any receptor that is not expressed/presented on the surface of resting macrophages in significant amounts could be used for targeting. The site that binds the ligand conjugates used in accordance with the present invention is a vitamin receptor: the folate receptor, which binds folate, or an analog or derivative thereof, according to claim 1.

In accordance with the invention the ligand conjugates can bind with high affinity to receptors on activated macrophages. The high affinity binding can be inherent to the ligand or the binding affinity can be enhanced by the use of a chemically modified ligand (i.e., an analog or a derivative) or by the particular chemical linkage, in the ligand conjugate, between the ligand and the immunogen, cytotoxin, or other compound capable of altering macrophage function.

The chemical linkage in the ligand conjugate between the ligand and the immunogen, can be a direct linkage or can be through an intermediary linker. If present, an intermediary linker can be any biocompatible linker known in the art. Typically, the linker comprises about 1 to about 30 carbon or other atoms, more typically about 2 to about 20 atoms. Lower molecular weight linkers (i.e., those having an approximate molecular weight of about 30 to about 300) are typically employed.

Generally, any manner of forming a conjugate between the ligand and the immunogen, between a linker and the ligand, or between a linker and the immunogen can be utilized in accordance with the present invention. With or without a linker, the complex can be formed by conjugation of the components of the conjugate, for example, through hydrogen, ionic, or covalent bonds. Covalent bonding of the components of the conjugate can occur, for example, through the formation of amide, ester, disulfide, or imino bonds between acid, aldehyde, hydroxy, amino, sulfhydryl, or hydrazo groups. Also, in accordance with this invention a linker can comprise an indirect means for associating the ligand with the immunogen, such as by connection through spacer arms or bridging molecules. The chemistry of the linker can be designed to resist lysis or become susceptible to lysis following uptake by the targeted macrophage. Both direct and indirect means for association should not prevent the binding of the ligand to the receptor on the activated macrophages for operation of the method of the present invention.

The folate ligand can be conjugated to the immunogen, by an art-recognized procedure that utilizes trifluoroacetic anhydride to prepare γ-esters of folic acid via a pteroyl azide intermediate. This procedure results in the synthesis of a folate ligand, conjugated to the immunogen only through the γ-carboxy group of the glutarnic acid groups of folate. Alternatively, folic acid analogs can he coupled by art-recognized procedures through the α-carboxy moiety of the glutamic acid group or both the a and γ carboxylic acid entities.

Alternatively, the ligand conjugate can be one comprising a liposome wherein the targeted entity is contained within a liposome which is itself covalently linked to the activated macrophage-binding ligand.

Treating lupus erythematosus may comprise administering to a patient suffering from such a disease state an effective amount of a composition comprising a conjugate of the formula L-X wherein L is as defined above and the group X comprises a cytotoxin.

Use of the conjugate of the present invention can result in an elimination or reduction of activated macrophages or other stimulated immune cells (e.g., stimulated dendritic cells or stimulated macrophages distinct from normal resting tissue resident macrophages) or an inhibition of the activity of the activated/stimulated cells. The conjugate may be used to treat symptomatic lupus erythematosus, The conjugate may also be used in a prophylactic treatment to prevent return of the symptoms of lupus erythematosus. The prophylactic treatment can comprise an initial treatment with the ligand conjugate, such as treatment in a multiple dose regimen, and one or more additional treatments or series of treatments after an interval of days or months following the initial treatment(s).

The group X in the activated macrophage targeted conjugate L-X comprises an immunogen, the ligand conjugates being effective to "label" a population of activated macrophages in the patient suffering from lupus erythematosus for specific elimination by an endogenous immune response or by co-administered antibodies. The use of ligand conjugates wherein X is an immunogen in accordance with this embodiment results in an immune response-mediated elimination of an activated macrophage population. Such can be effected through an endogenous immune response or by a passive immune response affected by co-administered antibodies. The endogenous immune response may include a humoral response, a cell-mediated immune response, and any other immune response endogenous to the patient, including complement-mediated cell lysis, antibody-dependent cell-mediated cytotoxicity (ADCC), antibody opsonization leading to phagocytosis, clustering of receptors upon antibody binding resulting in signaling of apoptosis, antiproliferation, or differentiation, and direct immune cell recognition of the delivered immunogen. It is also contemplated that the endogenous immune response will employ the secretion of cytokines that regulate such processes as the multiplication and migration of immune cells. The endogenous immune response may include the participation of such immune cell types as B cells, T cells, including helper and cytotoxic T cells, macrophages, natural killer cells, neutrophils, LAK cells, and the like.

In another embodiment, the ligand conjugates, wherein X is an immunogen, can be internalized and the immunogen can be degraded and presented on the macrophage cell surface for recognition by immune cells to elicit an immune response directed against macrophages presenting the degraded immunogen.

The humoral response can be a response induced by such processes as normally scheduled vaccination, or active immunization with a natural antigen or an unnatural antigen or hapten, e.g., fluorescein isothiocyanate (FITC) or dinitrophenyl (DNP), with the unnatural antigen inducing a novel immunity. Active immunization can involve multiple injections of the natural or unnatural antigen or hapten scheduled outside of a normal vaccination regimen to induce the immunity. The humoral response can also result from an innate immunity where the patient has a natural preexisting immunity, such as an immunity to α-galactosyl or foreign blood groups.

Alternatively, a passive immunity can be established by administering antibodies to the patient such as natural antibodies collected from serum or monoclonal antibodies that may or may not be genetically engineered antibodies, including humanized antibodies. The utilization of a particular amount of an antibody reagent to develop a passive immunity, and the use of a ligand conjugate, wherein X is an immunogen and wherein the passively administered antibodies are directed to the immunogen, would provide the advantage of a standard set of reagents to be used in cases where a patient's preexisting antibody titer is not therapeutically useful. The passively administered antibodies can be "co-administered" with the ligand conjugate, and co-administration is defined as administration of antibodies at a time prior to, at the same time as, or at a time following administration of the ligand conjugate.

In the above-described embodiments, wherein X in the ligand conjugate of the formula L-X is an immunogen, it is contemplated that the preexisting antibodies, induced antibodies, or passively administered antibodies can be redirected to activated macrophages by preferential binding of the ligand conjugates to the activated macrophage cell populations, and such cells can be killed by complement-mediated lysis, ADCC, antibody-dependent phagocytosis, or antibody clustering of receptors. The cytotoxic process may also involve other types of immune responses, such as cell-mediated immunity, as well as secondary responses that arise when the attracted antigen-presenting cells phagocytose the activated macrophages and present antigens of such cells to the immune system for elimination of other activated macrophages presenting such antigens.

Acceptable immunogens for use in preparing the conjugates used in the present invention arc immunogens that are capable of eliciting antibody production in the patient or that have previously elicited antibody production in the patient, resulting in a preexisting immunity, or that constitute part of the innate immune system. Alternatively, antibodies directed against the immunogen may be administered to the host animal to establish a passive immunity. Suitable immunogens for use in the invention include antigens or antigenic peptides against which a preexisting immunity has developed via normally scheduled vaccinations or prior natural exposure to such agents such as polio virus, tetanus, typhus, rubella, measles, mumps, pertussis, tuberculosis and influenza antigens, foreign blood group determinants, and α-galactosyl groups. In such cases, the ligand conjugates can be used to redirect a previously acquired humoral or cellular immunity to a population of activated macrophages in the patient for elimination of such cells.

Other suitable immunogens include antigens or antigenic peptides to which the patient has developed an immunity through immunization against a natural or an unnatural antigen or hapten, for example, fluorescein isothiocyanate (FITC) of dinitrophenyl (DNP) and antigens against which an innate immunity exists, for example, superantigens and muramyl dipeptide. It is also contemplated that MHC I restricted peptides could be linked to the ligand for use in redirecting cellular immunity to macrophages and eliciting T cell killing of macrophages.

At least one additional therapeutic factor can be administered to the patient in combination with the above-detailed methodology to enhance the elimination of activated macrophages, or more than one additional therapeutic factor can be administered. The therapeutic factor can be selected from a compound capable of stimulating an endogenous immune response, a cytotoxin, or another therapeutic factor capable of complementing the efficacy of the administered ligand conjugates. In addition to the above-described ligand conjugates, compounds or compositions capable of stimulating an endogenous immune response including cytokines or immune cell growth factors such as interleukins 1-18, stem cell factor, basic FGF, EGF, G-CSF, GM-CSF, FLK-2 ligand, HILDA, MIP-1α, TFG-α, TGD-β, M-CSF, IFN- a, IFN-β, IFN-γ, soluble CD23, LIF, and combinations thereof may be administered to the patient.

Chemotherapeutic agents, which are cytotoxic themselves and can kill or inhibit the activity of activated macrophages, suitable for use in the method of the invention in combination with the ligand conjugates include p38 MAP kinase inhibitors, adrcnocorticoids, alkylating agents, antiandrogens, antiestrogens, androgens, estrogens, antimetabolites such as cytosine arabinoside, purine analogs, pyrimidine analogs, and methotrexate, busulfan, carboplatin, chlorambucil, cisplatin and other platinum compounds, tamoxiphen, taxol, cyclophosphamide, plant alkaloids, prednisone, hydroxyurea, teniposide, antibiotics such as mitomycin C and bleomycin, nitrogen mustards, nitrosureas, vincristine, vinblastine, and any other art-recognized cytotoxic or chemotherapeutic agent. Alternatively, the ligand conjugates can be administered in combination with any other compound known to be useful for treatment of lupus erythematosus such as chloroquine.

The additional therapeutic factor can be administered to the patient prior to, after, or at the same time as the ligand conjugates and the therapeutic factor can be administered as part of the same composition containing the ligand conjugates or as part of a different composition than the ligand conjugates. Any such therapeutic composition containing an additional therapeutic factor at a therapeutically effective dose can be used in the method of the present invention. The therapeutic factor can be administered at a suboptimal dose along with the ligand conjugates in a combination therapy to avoid development of resistance to the therapeutic factor by the patient.

The amount of the ligand conjugate effective for use in accordance with the invention depends on many parameters, including the nature of the disease being treated, the molecular weight of the conjugate, its route of administration and its tissue distribution, and the possibility of co-usage of other therapeutic agents. The effective amount to be administered to a patient is typically based on body surface area, patient weight and physician assessment of patient condition. In accordance with the invention an "effective amount" of the ligand conjugate is an amount sufficient to bind to activated macrophages or other stimulated immune cells and to be useful in the treatment of lupus erythematosus. The effective amount of the ligand conjugate to be administered to a patient being treated for lupus erythematosus can range from, for example, about 1 ng/kg to about 10 mg/kg, or from about 10 µg/kg to about 1 mg/kg, or from about 100 µg/kg to about 500 µg/kg.

Any effective regimen for administering the ligand conjugates can be used. For example, the ligand conjugates can be administered as single doses, or they can be divided and administered as a multiple-dose daily regimen. Further, a staggered regimen, for example, one to three days per week can be used as an alternative to daily treatment, and for the purpose of defining this invention such an intermittent or staggered daily regimen is considered to be equivalent to every day treatment. A patient may be treated with multiple injections of the ligand conjugate to eliminate the population of activated macrophages. The patient may be treated, for example, injected multiple times with the ligand conjugate at, for example, at 12-72 hour intervals or at 48-72 hour intervals. Additional injections of the ligand conjugate can be administered to the patient at intervals of days or months after the initial injections, and the additional injections prevent recurrence of disease. Alternatively, the ligand conjugates can be administered prophylactically to prevent the occurrence of disease in patients known to be disposed to development of lupus erythematosus.

In one embodiment, more than one type of ligand conjugate can be used, for example, the patient can be pre-immunized with fluorescein isothiocyanate and dinitrophenyl and subsequently treated with fluorescein isothiocyanate and dinitrophenyl linked to the same or different targeting ligands in a co-dosing protocol. Alternatively, more than one type of ligand can be linked to one or more targeted entities (e.g., an immunogen) and conjugates with different ligands can be used in a co-dosing protocol.

The ligand conjugates administered in accordance with this invention are preferably administered parenterally to the patient being treated for lupus erythematosus, for example, intravenously, intradermally, subcutaneously, intramuscularly, or intraperitoneally, in combination with a pharmaceutically acceptable carrier. Alternatively, the conjugates can be administered to the patient being treated for lupus erythematosus by other medically useful procedures such as in an orally available formulation. In accordance with the invention a "patient being treated for lupus erythematosus" means any patient suspected of having lupus erythematosus who would be predicted to benefit from the method of the present invention.

The conjugates used in accordance with this invention of the formula L-X may be used to formulate diagnostic compositions comprising effective amounts of the conjugate and an acceptable carrier therefor. Examples of parenteral dosage forms include aqueous solutions of the conjugate, for example, a solution in isotonic saline, 5% glucose or other well-known pharmaceutically acceptable liquid carriers such as alcohols, glycols, esters and amides. The parenteral compositions can be in the form of a reconstitutable lyophilizate comprising the one or more doses of the ligand conjugate. Any orally available dosage forms known in the art can also be used.

The ligands conjugates can also be delivered to a patient using an osmotic pump. The ligand conjugates can he formulated as one of any of a number of prolonged release dosage forms known in the art such as, for example, the biodegradable carbohydrate matrices described in U.S. Patents Nos. 4, 713,249; 3,266,333; and 5,417,982 .

### EXAMPLE 1

### Analysis of EC20 Uptake in Organs of Lupus Prone MRL/MpJ-Tnfrsf6^{lpr} Mice

Lupus prone MRL/MpJ-*Tnfrsf6^{lpr}* mice were used to determine whether binding of a folate-targeted ^{99m}Tc chelating chemical moiety (EC20; see International Publication No. WO 03/092742) could be detected in the organs of mice prone to lupus erythematosus. Lupus prone MRL/MpJ-*Tnfrsf6^{lpr}* mice have a mutation within the gene encoding the Tnfrsf protein (a member of the tumor neurosis factor family), and at 3 months of age exhibit greatly increased levels of circulating immune complexes and severe glomerulonephritis, Female MRL/MpJ-*Tnfrsf6^{lpr}* mice have a 17-week life span. A control group of mice, denominated MRL/Mpl mice, are control mice that have only mild glomerular lesions at 3 months of age, and females have a 73-week life span.

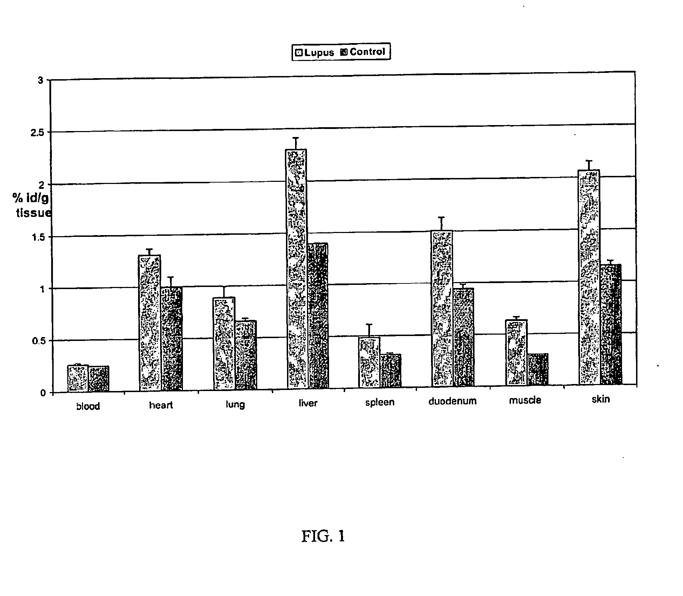

Female Lupus prone MRL/MpJ-*Tnfrsf6^{lpr}* mice (n = 3/group) and female MRL/MpJ mice (n = 3/group) were purchased from Jackson Laboratories (Bar Harbor, ME). The mice were placed on a folate deficient diet at the age of 12 weeks. Three weeks later the biodistribution of EC20 was evaluated in lupus prone and control mice. For the EC20 biodistribution studies, each mouse was administered with 1.8 mCi of ^{99m}Tc and 6.25 x 10⁻⁹ moles of EC20. The total injection volume for each mouse was 400 µl and the injections were i.p. The mice were sacrificed at 4 hours after injection, and organs were extracted for EC20 biodistribution analysis (i.e., the radioactivity per gram of tissue was measured (percentage injected dose of wet weight tissue (% ID/g)); see Fig. 1).

The results presented in Fig. 1 show that binding of ^{99m}Tc-EC20 was greater in all examined organs of lupus prone MRL/MpJ-*Tnfrsf6^{lpr}* mice compared to the organs of MRL/MpJ control mice. In particular, binding of ^{99m}Tc-EC20 was greater in the liver, spleen, duodenum, skin, and muscle of lupus prone MRL/MpJ-*Tnfrsf6*^{/*pr*} mice compared to control mice.

### EXAMPLE 2

### Effect of Folate-FITC Conjugates on Survival of Lupus Prone MRL/MpJ-Tnfrsf6^{lpr} Mice

Female lupus prone MRL/MpJ-*Tnfrsf6^{lpr}* mice were purchased from Jackson Laboratories (Bar Harbor, ME). Five-week old mice (n = 9/group) were immunized subcutaneously at multiple sites (2 sites in a 50 µl volume each) on day 0 with TiterMax Gold® adjuvant (in a 1:1 volume/volume ratio with folate-FITC conjugates) in combination with fluorescein isothiocyanate (FITC)-KLH conjugates (a total of 50 µg/mouse). Any effective adjuvant known in the art can be used. On day 20, the mice were placed on a folate deficient diet. On day 28 (at 9 weeks old) the mice were immunized a second time with TiterMax Gold® and fluorescein isothiocyanate (FITC)-KLH conjugates as described above.

After assuring that anti-FITC antibody titers were high in all mice (as evidenced by the results of ELISA assays of serum samples of the mice conducted on day 33), each animal was injected intraperitoneally at a single site with either phosphate buffered saline (PBS) or with 600 nmoles/kg of folate-FITC starting on day 35. For the next 25 weeks, the mice were injected daily with PBS or folate-FITC as described above.

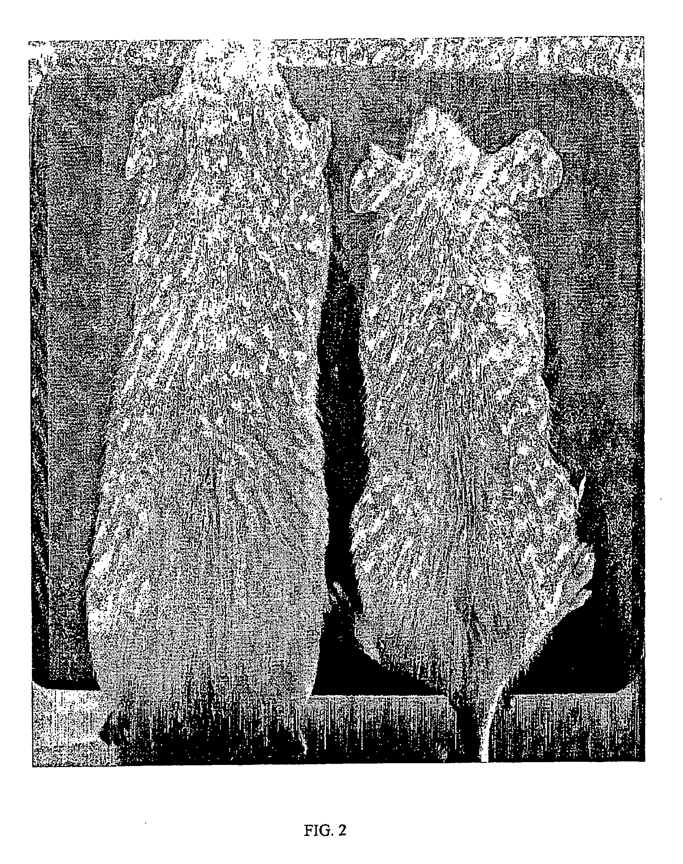

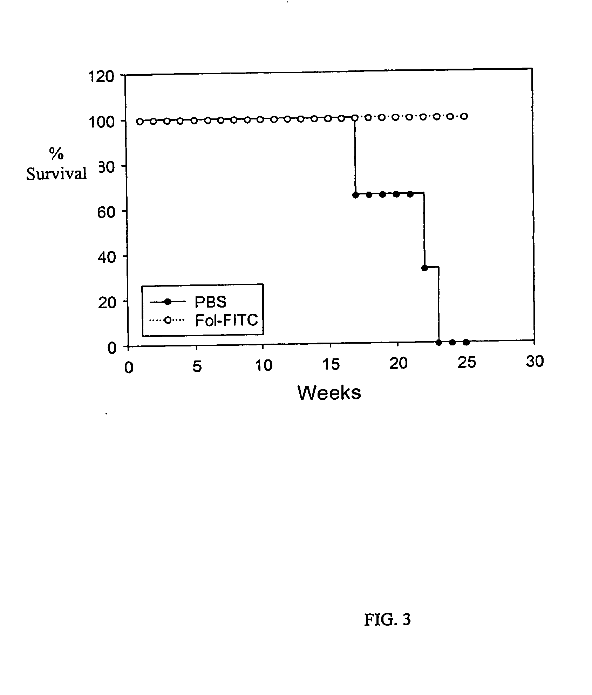

The efficacy of this immunotherapy was then evaluated by monitoring survival as a function of time of folate-FITC treated mice compared to control mice (i.e., injected with PBS). As shown in Fig. 3, control mice began dying at week 17 and all control mice were dead by week 25. In contrast, all mice treated with folate-FITC were alive at week 25 after initiation of treatment with folate-FITC conjugates indicating the method of the present invention is a promising treatment for lupus erythematosus. Fig. 2 shows a photomicrograph of lupus prone MRL/MpJ-*Tnfrsf6^{lpr}* mice from this study that were either injected with folate-FITC (mouse on the left) or were injected with PBS (control; mouse on the right). This photomicrograph shows the striking difference in the health of lupus prone mice that have been treated with folate-FITC conjugates versus lupus prone control mice.

## Claims

1. Use of a conjugate of the general formula
L-X
in the manufacture of a medicament for treating lupus erythematosus **characterised in that** the group L comprises a ligand capable of binding to activated macrophages and the group X comprises an immunogen and wherein the group L is selected from of folio acid, folinic acid, pteroic acid, pteropolyglutamic acid, a tetrahydropterin, a dihydrofolate, a tetrahydrofolate, a 1-deaza folate, a 3-deaza folate, a 5-deaza folate, an 8-deaza folate, a 10-deaza folate, a 1, 5 dideaza folate, a 5,10-dideaza folate, an 8,10-dideaza folate, a 5,8-dideaza folate, aminopterin, amethopterin, N10 -methylfolate, 2-deamino-hydroxyfolate, 1-deazamethopterin, 3-deazamethopterin, and 3'5'-dichloro-4-amino-4-deoxy-N 10-methylpteroylglutamic acid.

2. Use of claim 1 wherein the immunogen comprises fluorescein isothiocyanate.

3. Use of claim 1 wherein the immunogen comprises dinitrophenyl.

4. Use of claim 1 wherein the medicament is in a parenteral dosage form.

5. Use of claim 4 wherein the parenteral dosage form is selected from intravenous, intradermal, subcutaneous, intramuscular, and intraperitoneal dosage forms.

6. Use of claim 1 wherein the medicament further comprises a pharmaceutically acceptable carrier.

7. Use of claim 6 wherein the pharmaceutically acceptable carrier is a liquid.

8. Use of claim 7 wherein the liquid carrier is selected from alcohols, glycols, esters, amides, and a combination thereof.

9. Use of claim 7 wherein the liquid comprises an isotonic saline solution.

10. Use of claim 7 wherein the liquid comprises a 5% glucose solution.

11. Use of claim 5 wherein the conjugate within the medicament is lyophilized.

12. Use of claim 1 where the medicament is in an orally available dosage form.

13. Use of claim 1 wherein the medicament is administered to a patient in an effective amount and wherein the effective amount ranges from about 1 ng to about 10 mg per kilogram of body weight.

14. Use of claim 13 wherein the effective amount ranges from about 10 µg to about 1 mg per kilogram of body weight.

15. Use of claim 13 wherein the effective amount ranges from about 100 µg to about 500 µg per kilogram of body weight.

16. Use of claim 1 wherein the medicament is to be administered in a multiple-dose daily regimen.

17. Use of claim 1 wherein the medicament is to be administered prophylactically to a patient to prevent the occurrence of lupus erythematosus.

18. Use of claim 1 wherein the medicament is used in combination with at least one additional therapeutic factor.

19. Use of claim 18 wherein the additional therapeutic factor is a cytokine.

20. Use of claim 19 wherein the cytokine is selected from intaleukins 1-18, stem cell factor, basic FGF, EGF, G-CSF, CM-CSF, FLK-2 ligand, HILDA, MIP-α TGF-α TGF-β M-CSF, IFN-α IFN-β, IFN-γ soluble CD23, LIF, and a combination thereof.

21. A composition of the general formula
L-X
for use in treating lupus erythematosus **characterised in that** the group L comprises a ligand capable of binding to activated macrophages and the group X comprises an immunogen and wherein the group L is selected folic acid , folinic acid, pteroic acid, pteropolyglutamic acid, a tetrahydropterin, a dihydrofolate, a tetrahydrofolate, a 1-deaza folate, a 3-deaza folate, a 3-deaza folate, an 8-deaza folate, a 10-deaza folate, a 1,5 dideaza folate, a 5,10-dideaza folate, an 8,10-dideaza folate, a 5,8-dideaza folate, aminopterin, amethopterin, N10 -methylfolate, 2-deamino-hydroxyfolate, 1-deazamethopterin, 3-deazamethopterin, and 3'5'-dichloro-4-amino-4-deoxy-N10-methylpteroylglutamic acid.

## Patentansprüche

1. Verwendung eines Konjugats der allgemeinen Formel
L-X
in der Herstellung eines Medikaments zur Behandlung von Lupus Erythematodes, **dadurch gekennzeichnet, dass** die Gruppe L einen Liganden umfasst, der an aktivierte Makrophagen binden kann, und die Gruppe X ein Immunogen umfasst, und wobei die Gruppe L ausgewählt ist aus Folsäure, Folinsäure, Pteroinsäure, Pteropolyglutaminsäure, einem Tetrahydropterin, einem Dihydrofolat, einem Tetrahydrofolat, einem 1-Deazafolat, einem 3-Deazafolat, einem 5-Deazafolat, einem 8-Deazafolat, einem 10-Deazafolat, einem 1,5-Dideazafolat, einem 5,10-Dideazafolat, einem 8,10-Dideazafolat, einem 5,8-Dideazafolat, Aminopterin, Amethopterin, N10-Methylfolat, 2-Deamino hydroxyfolat, 1-Deazamethopterin, 3-Deazamethopterin und 3'5'-Dichloro-4-amino-4-deoxy-N₁₀-Methylpteroylglutaminsäure.

2. Verwendung gemäß Anspruch 1, wobei das ImmunogenFluorescein-Isothiocyanat umfasst.

3. Verwendung gemäß Anspruch 1, wobei das ImmunogenDinitrophenyl umfasst.

4. Verwendung gemäß Anspruch 1, wobei das Medikament in parenteraler Darreichungsform vorliegt.

5. Verwendung gemäß Anspruch 4, wobei die parenterale Darreichungsform ausgewählt ist aus intravenösen, intradermalen, subkutanen, intramuskulären und intraperitonealen Darreichungsformen.

6. Verwendung gemäß Anspruch 1, wobei das Medikament weiter einen pharmazeutisch annehmbaren Träger umfasst.

7. Verwendung gemäß Anspruch 6, wobei der pharmazeutisch annehmbare Träger eine Flüssigkeit ist.

8. Verwendung gemäß Anspruch 7, wobei der flüssige Träger ausgewählt ist aus Alkoholen, Glykolen, Estern, Amiden, und einer Kombination davon.

9. Verwendung gemäß Anspruch 7, wobei die Flüssigkeit eine isotone Kochsalzlösung umfasst.

10. Verwendung gemäß Anspruch 7, wobei die Flüssigkeit eine 5% Glukoselösung umfasst.

11. Verwendung gemäß Anspruch 5, wobei das Konjugat innerhalb des Medikaments lyophilisiert ist.

12. Verwendung gemäß Anspruch 1, wobei das Medikament eine oral verfügbare Darreichungsform ist.

13. Verwendung gemäß Anspruch 1, wobei das Medikament an einem Patienten in einer effektiven Menge verabreicht wird, und wobei die effektive Menge im Bereich von ungefähr 1 ng bis ungefähr 10 mg pro Kilogramm Körpergewicht liegt.

14. Verwendung gemäß Anspruch 13, wobei die effektive Menge im Bereich von ungefähr 10µg bis ungefähr 1mg pro Kilogramm Körpergewicht liegt.

15. Verwendung gemäß Anspruch 13, wobei die effektive Menge im Bereich von ungefähr 100 µg bis ungefähr 500 µg pro Kilogramm Körpergewicht liegt.

16. Verwendung gemäß Anspruch 1, wobei das Medikament im Schema einer täglichen Mehrfachgabe verabreicht werden soll.

17. Verwendung gemäß Anspruch 1, wobei das Medikament prophylaktisch an einen Patienten verabreicht werden soll, um das Auftreten von Lupus Erythematodes zu verhindern.

18. Verwendung gemäß Anspruch 1, wobei das Medikament in Kombination mit zumindest einem zusätzlichen therapeutischen Faktor verwendet wird.

19. Verwendung gemäß Anspruch 18, wobei der zusätzliche therapeutische Faktor einCytokin ist.

20. Verwendung gemäß Anspruch 19, wobei das Cytokin ausgewählt ist aus Interleukinen 1-18, Stammzellenfaktor, allgemeinem FGF, EGF, G-CSF, GM-CFS, FLK-2 Ligand, HILDA, MIP-Iα, TGF-α, TGF-β, M-CSF, IFN-α, IFN-β, IFN-γ, lösliches CD₂₃, LIF, und einer Kombination davon.

21. Eine Zusammensetzung der allgemeinen Formel
L-X
zur Verwendung in der Behandlung von Lupus Erythematodes, **dadurch gekennzeichnet, dass** die Gruppe L einen Ligand umfasst, der an aktivierte Makrophagen binden kann, und die Gruppe X ein Immunogen umfasst, und wobei die Gruppe L ausgewählt ist aus Folsäure, Folinsäure, Pteroinsäure, Pteropolyglutaminsäure, einem Tetrahydropterin, einem Dihydrofolat, einem Tetrahydrofolat, einem 1-Deazafolat, einem 3-Deazafolat, einem 5-Deazafolat, einem 8-Deazafolat, einem 10-Deazafolat, einem 1,5-Dideazafolat, einem 5,10-Dideazafolat, einem 8,10-Dideazafolat, einem 5,8-Dideazafolat, Aminopterin, Amethopterin, N10-Methylfolat, 2-Deamino hydroxyfolat, 1-Deazamethopterin, 3-Deazamethopterin und 3'5'-Dichloro-4-amino-4-deoxy-N10-Methylpteroylglutaminsäure.

## Revendications

1. Utilisation d'un conjugué ayant la formule générale
L-X
pour la production d'un médicament pour traiter le lupus érythémateux **caractérisée en ce que** le groupe L comprend un ligand capable de se lier à des macrophages activés et que le groupe X comprend un immunogène et dans lequel le groupe L est sélectionné parmi l'acide folique, l'acide folinique, l'acide ptéroïque, l'acide ptéropolyglutamique, une tétrahydroptérine, une dihydrofolate, un tétrahydrofolate, un folate 1-déaza, un folate 3-déaza, un folate 5-déaza, un folate 8-déaza, un folate 10-déaza, un folate 1,5-didéaza, un folate 5,10-didéaza, un folate 8,10-didéaza, un folate 5,8-didéaza, l'aminoptérine, l'améthoptérine, le folate de N10-méthyle, le 2-désamino-hydroxyfolate, la 1-déazaméthoptérine, la 3-déazaméthoptérine et l'acide 3'5'-dichloro-4-amino-4-désoxy-N10-méthylptéroylglutamique.

2. Utilisation selon la revendication 1 dans laquelle l'immunogène comprend de l'isothiocyanate de fluorescine.

3. Utilisation selon la revendication 1 dans laquelle l'immunogène comprend du dinitrophényle.

4. Utilisation selon la revendication 1 dans laquelle le médicament se présente sous une forme pharmaceutique parentérale.

5. Utilisation selon la revendication 4 dans laquelle la forme pharmaceutique parentérale est sélectionnée parmi des formes pharmaceutiques intraveineuses, intradermiques, sous-cutanées, intramusculaires et intrapéritonéales.

6. Utilisation selon la revendication 1 dans laquelle le médicament comprend de plus un excipient pharmaceutiquement acceptable.

7. Utilisation selon la revendication 6 dans laquelle l'excipient pharmaceutiquement acceptable est un liquide.

8. Utilisation selon la revendication 7 dans laquelle l'excipient liquide est sélectionné parmi des alcools, des glycols, des esters, des amides et une combinaison de ceux-ci.

9. Utilisation selon la revendication 7 dans laquelle le liquide comprend une solution saline isotonique.

10. Utilisation selon la revendication 7 dans laquelle le liquide comprend une solution de glucose à 5 %.

11. Utilisation selon la revendication 5 dans laquelle le conjugué se trouvant dans le médicament est lyophilisé.

12. Utilisation selon la revendication 1 dans laquelle le médicament se présente sous une forme pharmaceutique compatible avec l'administration par voie orale.

13. Utilisation selon la revendication 1 dans laquelle le médicament est administré à un patient à une quantité efficace et dans laquelle la quantité efficace va de 1 ng environ à 10 mg environ par kilogramme de poids corporel.

14. Utilisation selon la revendication 13 dans laquelle la quantité efficace va de 10 µg environ à 1 mg environ par kilogramme de poids corporel.

15. Utilisation selon la revendication 13 dans laquelle la quantité efficace va de 100 µg environ à 500 µg environ par kilogramme de poids corporel.

16. Utilisation selon la revendication 1 dans laquelle le médicament doit être administré selon un régime posologique comprenant plusieurs doses quotidiennes.

17. Utilisation selon la revendication 1 dans laquelle le médicament doit être administré à un patient à titre de prophylaxie afin de prévenir l'apparition du lupus érythémateux.

18. Utilisation selon la revendication 1 dans laquelle le médicament est utilisé en association avec au moins un facteur thérapeutique supplémentaire.

19. Utilisation selon la revendication 18 dans laquelle le facteur thérapeutique supplémentaire est une cytokine.

20. Utilisation selon la revendication 19 dans laquelle la cytokine est sélectionnée parmi les interleukines 1-18, le facteur de cellules souches, les facteurs de base FGF, EGF, G-CSF, GM-CSF, le ligand FLK-2, HILDA, MIP-1α, TGF-α TGF-β M-CSF, IFN-α IFN-β IFN-γ CD23 soluble, LIF et une combinaison de ceux-ci.

21. Composition ayant la formule générale
L-X
destinée à être utilisée pour traiter le lupus érythémateux **caractérisée en ce que** le groupe L comprend un ligand capable de se lier à des macrophages activés et que le groupe X comprend un immunogène et dans laquelle le groupe L est sélectionné parmi l'acide folique, l'acide folinique, l'acide ptéroïque, l'acide ptéropolyglutamique, une tétrahydroptérine, une dihydrofolate, un tétrahydrofolate, un folate 1-déaza, un folate 3-déaza, un folate 5-déaza, un folate 8-déaza, un folate 10-déaza, un folate 1,5-didéaza, un folate 5,10-didéaza, un folate 8,10-didéaza, un folate 5,8-didéaza, l'aminoptérine, l'améthoptérine, le folate de N10-méthyle, le 2-désamino-hydroxyfolate, la 1-déazaméthoptérine, la 3-déazaméthoptérine et l'acide 3'5'-dichloro-4-amino-4-désoxy-N10-méthylptéroylglutamique.
